# EUROPEAN PATENT SPECIFICATION

(11) **EP 1 734 946 B1**
(45) Date of publication and mention of the grant of the patent: **27.06.2012**
(21) Application number: 05711709.5
(22) Date of filing: 20.01.2005
(51) Int. Cl.: A61K 31/202, A61K 31/232, A61P 37/04, A61P 31/16, A61P 1/00, A61P 29/00, A61P 3/04, A61P 3/10, A61P 31/12, A61P 31/04

(54) **Punicic acid for use to enhance immune response and treating inflammatory bowel disease**
Punicin-Säure zur Verbesserung der Immunantwort und Behandlung von entzündlicher Darmerkrankung
Acide punicique pour améliorer la réponse immunitaire et traiter les maladies intestinales inflammatoires

(30) Priority: 20.01.2004 US 537617 P; 07.01.2005 US 31591; 07.01.2005 WO PCT/US2005/000581
(43) Date of publication of application: 27.12.2006
(73) Proprietor: Nutrition Therapeutics, Inc., Blacksburg VA 24060-0108 (US)
(72) Inventor: BASSAGANYA-RIERA, Josep, Blacksburg, VA 24060 (US)
(74) Representative: Lorente Berges, Ana
(86) International application number: PCT/US2005/001811
(87) International publication number: WO 2005/070412

(56) References cited:
- EP-A- 1 175 901
- EP-A- 1 437 412
- WO-A-99/66941
- SCHUBERT S Y ET AL: "ANTIOXIDANT AND EICOSANOID ENZYME INHIBITION PROPERTIES OF POMEGRANATE SEED OIL AND FERMENTED JUICE FLAVONOIDS" JOURNAL OF ETHNOPHARMACOLOGY, ELSEVIER SCIENTIFIC PUBLISHERS LTD, IE, vol. 66, no. 1, 1999, pages 11-17, XP000946139 ISSN: 0378-8741
- NUGTEREN D H ET AL: "NATURALLY OCCURRING CONJUGATED OCTADECATRIENOIC ACIDS ARE STRONG INHIBITORS OF PROSTAGLANDIN BIOSYNTHESIS" PROSTAGLANDINS, BUTTERWORTH, STONEHAM, MA, US, vol. 33, no. 3, March 1987 (1987-03), pages 403-417, XP002063858 ISSN: 0090-6980
- BASSAGANYA-RIERA J ET AL: "Colonic anti-inflammatory mechanisms of conjugated linoleic acid." CLINICAL NUTRITION (EDINBURGH, LOTHIAN) DEC 2002, vol. 21, no. 6, December 2002 (2002-12), pages 451-459, XP008046644 ISSN: 0261-5614
- TAKENAGA M ET AL: "IN VITRO EFFECT OF TRICHOSANIC ACID, A MAJOR COMPONENT OF TRICHOSANTHES JAPONICA ON PLATELET AGGREGATION AND ARACHIDONIC ACID METABOLISM IN HUMAN PLATELETS" PROSTAGLANDINS, LEUKOTRIENES AND MEDICINE, CHURCHILL LIVINGSTONE, EDINBURGH, NEW YORK,, GB, vol. 31, no. 2, 1988, pages 65-72, XP008046646 ISSN: 0262-1746
- DATABASE CA [Online] CHEMICAL ABSTRACTS SERVICE, COLUMBUS, OHIO, US; AKIHISA, TOSHIHIRO ET AL: "Carcinogenesis-preventing agents containing fatty acids and/or their methyl esters" XP002328421 retrieved from STN Database accession no. 2004:17835 & JP 2004 002269 A2 (NIHON UNIVERSITY, JAPAN) 8 January 2004 (2004-01-08)
- DE M ET AL: "Antimicrobial screening of some Indian spices." PHYTOTHERAPY RESEARCH : PTR. NOV 1999, vol. 13, no. 7, November 1999 (1999-11), pages 616-618, XP008047201 ISSN: 0951-418X
- DATABASE WPI Section Ch, Week 200303 Derwent Publications Ltd., London, GB; Class B04, AN 2003-032454 XP002344249 & JP 2002 176913 A (KANEKA CORP) 25 June 2002 (2002-06-25)
- DATABASE CA [Online] CHEMICAL ABSTRACTS SERVICE, COLUMBUS, OHIO, US; 11 June 2002 (2002-06-11), SHIRAISHI, TADAYOSHI ET AL: "Processed fats and oils containing conjugated trienoic acid glycerides and foods using the fats and oils" XP002343727 retrieved from STN Database accession no. 2002:438844 & JP 2002 165559 A2 (KANEGAFUCHI CHEMICAL INDUSTRY CO., LTD., JAPAN) 11 June 2002 (2002-06-11)
- BOUSSETTA TAREK ET AL: "Punicic acid a conjugated linolenic acid inhibits TNFalpha-induced neutrophil hyperactivation and protects from experimental colon inflammation in rats.", PLOS ONE 2009 LNKD- PUBMED:19649246, vol. 4, no. 7, 2009, page e6458, ISSN: 1932-6203

## Description

### FIELD OF THE INVENTION

The present invention is generally directed to the use of punicic acid to treat, prevent or/and ameliorate inflammatory Bowel Disease (IBD)

### CROSS-REFERENCE TO RELATED APPLICATION

This application claims priority under 35 U.S.C. §119(e) to U.S. Provisional Application Serial No. 60/537,617 filed 20 January 2004.

### CITED REFERENCES

A full bibliographic citation of the references cited in this application can be found in the section preceding the claims.

### DEFINITIONS

The following definitions are used throughout present application:
Antigen: Any substance that may be specifically bound by an antibody molecule or T cell receptor.
ANOVA: Analysis of variance. Arithmetic process for partitioning the overall variation in data sets into specific components based on sources of variation. It has been used to determine whether numerical differences between treatment groups are statistically significant.
Adipogenesis: the process by which new adipocytes or fat storage cells are generated.
CD19: Cluster Differentiation 19. A protein expressed on the surface of B cells.
CD4: Cluster Differentiaion 4. A co-receptor found on the surface of helper T cells.
CD3: Cluster Differentiation 3. A co-receptor found on the surface of all T cells.
CD8: Cluster Differentiation 8. A co-reccptor found on the surface of cytotoxic T cells.
CD4⁺ T cells: Helper T cells. Cells that secrete proteins that modulate the activity of other immune cells against antigens.
CD8⁺ T cells: Cytotoxic T cells. Cells capable of disabling or destroying abnormal cells, including tumor cells, virus-infected cells and cells infected with intracellutar bacteria.
Con A: Concanavalin A. A mitogen that induces proliferation of lymphocytes. Stimulation of lymphocytes with ConA has been widely utilized to examine the effects of dietary interventions on immune function.
cRPMI: Complete Roswell Park Memorial Institute media. A cell culture media formulated to contain all the nutrients necessary for long-term cell survival. Detailed composition shown under media and reagent preparation.
DSS: Dextran Sodium Sulfate. A chemical administered by drinking water (2.5% wt/v) to cause the death of the epithelial cells of the colon and induces colonic inflammation. The DSS colitis model is a well-established model of experimental IBD.
Epitope: A part of an antigen presented in the major histocompatibility complex **I** or 2 and that is recognized specifically either by an antibody or by the T cell receptor.
FACS: Fluorescence-Activated Cell Sorting. A special buffer used for flow cytometry applications to prevent the internalization of cell surface markers. Detailed composition shown under media and reagent preparation.
Glycemia: concentration of glucose in blood.
Hyperglycemia: increase concentrations of glucose in blood beyond the normal ranges.
Hyperinsulinemia: increased concentrations of insulin in blood beyond the normal ranges.
IBD: Inflammatory Bowel Disease. An immunoinflammatory disease of the intestine characterized by two clinical manifestations - Crohn's Disease (CD) and Ulcerative Colitis (UC).
Insulinemia: concentration of insulin in blood.
mAb: Monoclonal antibody. An antibody produced by B cells in response to specific antigenic stimulation that binds to specific regions of a protein.
Nutraceutical: A compound with specific medicinal as well as nutritional benefits.
PBS: Phosphate-Buffered Saline. A buffer used in cell culture applications. Cells can be resuspended in PBS for short periods of time before transfer to a more nutritive butter. Detailed composition shown under media and reagent preparation.
TCR: T-Cell Receptor. A protein expressed on the surface of T lymphocytes, co-expressed always with the CD3 molecule and that recognizes specific antigens. Following antigen recognition, it activates the lymphocyte and initiates an immune response against the antigen.

Type 2 diabetes or Non-insulin dependent Diabetes Mellitus. It represents the more common type of diabetes. It is caused by an unresponsiveness of cells to the actions of insulin. If the cells do not respond to insulin, they are unable to take up glucose from blood, which results in glucotoxicity. In addition, the cells are deprived from the energy derived from glucose oxidation.

### DESCRIPTION OF THE PRIOR ART

The immune response is the mechanism of defense against foreign substances that invade to cause infection and/or disease. The immune system's functions are complicated processes that involve the coordinated efforts of several types of cells, including white blood cells. The immune response begins when an antigen-presenting cell encounters a foreign antigen. The antigen-presenting cell degrades it and displays pieces of the antigen (i.e., viral, bacterial or of other origin called epitopes on its surface.

Unique among the many different clones of T cells (a subpopulation of white blood cells) in the body, one particular T cell clone recognizes the antigen displayed and binds to the antigen-presenting cell. This union modulates the production of proteins called cytokines by the antigen-presenting cell, such as interieukin-1 (IL-1) and tumor necrosis factor (TNF). These proteins are used in the communication between the antigen-presenting cell and T cell. The T cell also produces other proteins such as interleukin-2 (IL-2) and gamma interferon (IFN-0. Together, the T cell and antigen-presenting cell facilitate intercellular communication. As part of the continuing process, IL-2 instructs other T cells and killer T cells to multiply.

The proliferating helper T cells release substances that cause B cells to multiply and produce antibodies. The antibodies released by the B cells bind to antigens on the surfaces of free-floating viruses and other pathogens. As the infection is brought under control, the activated T and B cells are turned off by suppressor T cells. However, a few "memory cells" remain behind to respond quickly if the same pathogen attacks again.

Researchers believe that the mammalian immune system fights all antagonists in much the same way as described above. However, further study is needed to reveal novel ways to bolster the immune system to enhance the immune response of an animal. Ideally, these novel ways of enhancing the immune system should also prevent or attenuate the adverse side effects associated with immune and/or inflammatory responses.

Current research also Indicates that other immune disorders such as Inflammatory Bowel Disease (IBD) may also be helped by further research into the immune system. IBD is a chronic, recurring immunoinflammatory illness of unknown etiology afflicting over 1,000,000 Americans and several million people worldwide. IBD is a prevalent cause of chronic illness in a large segment of the patient population. It can manifest itself in two different forms: Ulcerative Colitis (UC) and Crohn's Disease (CD). Although the two conditions clinically appear very similar, UC primarily involves inflammation of the colon and rectum, as opposed to the upper gastrointestinal tract. CD, on the other hand, impacts a greater area of the upper intestinal digestive tract in an animal, and is thus more likely to trigger malabsorption, along with chronic vitamin and nutrient deficiencies.

Individuals suffering from IBD experience symptoms characterized by chronic intestinal inflammation that results in clinical symptoms such as diarrhea, bleeding, abdominal pain, fever, joint pain, and weight loss. These symptoms can range from mild to severe. IRD may gradually and subtly develop from an initial minor discomfort, or may present suddenly with acute intensity.

Current treatments for IBD include corticosteroids such as 6-methylprednisolone and budesonide, and immunosuppressives such as azathioprine, 6-mercaptopurine, cyclosporine, and methotrexate, (Lichtenstein et al. 2003). New therapies still under development include the Food and Drug Administration-approved anti-tumor necrosis factor (TNF)-∀ (Camilleri 2003; Lichtenstein et al. 2003; Bassaganya-Riera et al. 2004). Clinical Nutrition (2002), 21(6): p. 451-9 discloses the beneficial effects of conjugated linoleic acid on the susceptibility to IBD. While existing therapies against IBD have improved, they remain only modestly successful. Further, conventional therapies often result in significant side effects to the user. Therefore, there remains a need for novel preventive and/or therapeutic methods for treating immunoinflammatory diseases such as IBD.

Punicic acid is a non-toxic, natural, orally active food ingredient known to humans and consumed by humans for centuries. Punicic acid is naturally found in seeds of pomegranate, i.e., *Punica granatum,* representing over 60 percent of the oil. *Momordica balsamina* is another medicinal plant belonging to the cucumber family with a similar concentration of punicic acid. The presence of punicic acid in pomegranate seeds and other medicinal plants is well-known in the field. Also, the benefits of pomegranates against cancer are well known in the field. For instance, methods of treating medical disorders using the pomegranate fruit are common. However, methods for treating IBD in mammals using punicic acid are not.

For instance, U.S. Patent 6,630,163 to Murad teaches a method of treating dermatological disorders with fruit extracts, including pomegranate. The method includes administering therapeutically effective amounts of at least one fruit extract to neutralize free radicals in the skin. This method is aimed at targeting and curing disorders in the skin.

U.S. Patent 6,030,622 to Shehadeh teaches a method of preparing an herbal extract composition comprising extract of arum, extract of pomegranate, extract of tea and extract of hibiscus. The components of the pomegranate used to create the extract are fruit peels. The fruit peels do not contain punicic acid because the punicic acid is only found in the seed. In addition, the methods of extraction utilized (aqueous and ethanolic) are designed to extract hydrosoluble components, not the oil or the punicic acid.

Further, U.S. Patents 6,060,063 and 5,891,440 to Lansky teach a method of preparing a phytoestrogen oral supplement and ointment by extracting pomegranate seeds with an aqueous solvent and admixing the pomegranate seed extract with an herbal extract containing shizandra berries, Chinese asparagus root, and optionally Chinese licorice and Chinese angelica root.

Japanese Patent 2002238566 to Murase and Imamura teaches a method of cloning the gene involved in the synthesis of catalpic and punicic acid.

Other patents, such as Great Britain Patents 1466418 and 758724 teach methods of polymerizing conjugated trienoic fatty acids or esters and improving synthetic resins using punicic acid.

Further, while the use of pomegranate seed oil has been shown to be useful against the proliferation and survival of human breast adenocarcinoma (MCF-7) cells, only in-vitro studies using topical administrations have been completed (Kim et al. 2002). Other studies have shown the use of pomegranate oil to be effective in the treatment of tumors in a mouse model of chemically induced skin cancer (Hora et al. 2003).

### SUMMARY OF THE INVENTION

In response to the above-described needs, the present invention deals with punicic acid, esters thereof, pharmaceutically suitable salts thereof, to treat Inflammatory Bowel Disease (IBD). Punicic acid also shows promise in adjunct therapies aimed at further enhancing the efficacy of other pharmacologic treatments currently utilized to prevent or ameliorate IBD.

While any of the punicic acid forms may be used, in a preferred embodiment, the free acid form of punicic acid is used.

In a preferred embodiment of the present invention, the punicic acid compound is administered orally to the animal. The punicic acid compound may be administered alone or in combination with a pharmaceutically suitable carrier.

The punicic acid compound may also be administered parenterally, via injection or rectally. The punicic acid compound may be administered alone or in combination with a pharmaceutically suitable excipient.

In another embodiment of the present invention, a therapeutically effective amount of the punicic acid compound is administered to an animal in combination with a nutritional food supplement. Such supplements include but are not limited to infant formulas, children products, geriatric formulas, milk, cheese, kefir, cereal bars, weight management formulas, energy bars, other human foods, functional foods, and animal feed.

Punicic acid may also be administered in combination with other active ingredients such as vitamins or other fatty acids.

The effective amount of the punicic acid compound depends on the needs of the animal

In another embodiment, an amount of punicic acid compound effective to treat Inflammatory Bowel Disease (IBD) is administered to the animal. Specifically, an amount effective to prevent or ameliorate the clinical signs and intestinal lesions associated with IBD is administered.

In yet another embodiment, an amount of punicic acid compound effective to increase CD4⁺ and CD8⁺ T lymphocyte levels in an animal is administered. The amount of punicic acid compound is preferably effective to increase T cell levels while preventing exacerbated immune responses, allergies, hypersensitivity reactions, or autoimmune reactions.

The invention also provides the use of a punicic acid in the manufacture of a composition for preventing or ameliorating an intestinal immune or inflammatory disorder.

The application also discloses a method of improving the nutritional quality of infant formulas, geriatric formulas, milk, cheese, kefir, cereal bars, weight management formulas, other human foods and animal feed, comprising the step of adding a safe and effective dose of punicic acid in the formula's composition.

The formulations of punicic acid disclosed in the present invention may be conveniently presented in unit dosage form and may be prepared by any methods well known in the art of pharmacy or nutrition. Possible formulations include but are not limited to capsules, cachets, tablets, boluses or lozenges, each containing a predetermined amount of punicic acid.

Advantageously, there is no upper limit to the amount of punicic acid that may be administered to an animal in need thereof. Further, the method of the present invention may be administered to animals, including mammals and humans, of all ages and health. For instance, vulnerable populations such as the elderly, obese, diabetic, sick or very young can benefit from the present invention, as can healthy individuals with no bistoury of immunosuppression.

The scope of the invention will appear more fully from the following detailed description of the preferred embodiment of the invention made in conjunction with the accompanying drawings.

### BRIEF DESCRIPTION OF THE DRAWINGS

Figure 1A is a graph illustrating the effects of dietary punicic acid and an isocaloric control diet on body weight losses during Experiment 1.
Figure 1B is a graph illustrating the effects of dietary punicic acid and an isocaloric control diet on rectal bleeding during Experiment 1.
Figure 1C is a graph illustrating the effects of dietary punicic acid and an isocaloric control diet on disease activity indices during Experiment 1.
Figure 2A is a photomicrograph of hematoxylin and eosin (H&E)-stained on paraffin-embedded colonic tissue recovered from mice fed the punicic acid during Experiment 1 at 20x magnification.
Figure 2B is a photomicrograph of H&E-stained on paraffin-embedded colonic tissue recovered from mice fed the punicic acid diet during Experiment I. at 20x magnification.
Figure 2C is a photomicrograph of H&E-stained on paraffin-embedded colonic tissue recovered from mice fed the control diet during Experiment 1.
Figure 2D is a photomicrograph ofH&E-stained on paraffin-embedded colonic tissue recovered from mice fed the control diet during Experiment 1.
Figure 2E is a photomicrograph of H&E-stained on paraffin-embedded colonic tissue recovered from mice fed the punicic acid diet during Experiment 1 at 40x magnification.
Figure 2F is a photomicrograph of H&E-stained on paraffin-embeddod colonic tissue recovered from mice fed the punicic acid diet during Experiment 1 at 40x magnification.

### DETAILED DESCRIPTION OF THE INVENTION

### Punicic Acid

The term, as used herein, refers to a conjugated linolenic acid isomer containing cis-9, trans-11, cis-13 double bonds in the C₁₈ carbon chain, its non-toxic salts, active esters, active isomers, active metabolites, structural lipids containing punicic acid, and mixtures thereof. Punicic acid is also known also as trichosanic acid and is found in the seed oil of Punica granatum (Punicaceae, Pomegranate) and Trichosanthes anguina (Cucurbitaceae, snake gourd). Punicic acid constitutes approximately 86% of the oil of the pomegranate seed Non-tnxic salts include, for example, alkyl esters having from 1 to 6 carbon atoms in the alkyl group, as well as mono-, di- and tri- glycerides, and mixtures thereof. Active isomers of punicic acid include geometrical isomers such as eleostearic acid (cis-9, trans-11, trans-13 octadecatrienoic acid) and its non-toxic salts (e.g., sodium, potassium, calcium and magnesium salts) and its active esters (e.g., alkyl esters having from 1 to 6 carbon atoms in the alkyl group, as well as mono-, di- and tri- glycerides, and mixtures thereof.

The punicic acid may be a substantially pure single chemical compound or a mixture of one or more punicic acid compounds as defined above. The term substantially pure means having a purity of at least 90% by weight, preferably at least 95 % by weight such as at least 98%, 99% or about 100% by weight. For example, the punicic acid may be in the form of an extract obtainable or obtained from pomegranate seed oil, either directly or following one or more steps of purification.

Punicic acid has an extremely strong ability to resist the oxidizing, inflammation and destruction functions of the free radical of oxygen. Punicic acid has been shown to prevent scleratheroma as well as to delay the progression of cancer in the body. Punicic acid acts physiologically as an antioxidant also acts to lower plasma cholesterol. Further, punicic acid has an inhibitory effect in vitro on aggregation and arachidonic acid metabolism in human platelets. Based on just these few characteristics, punicic acid has wide application prospects in medicines and health protection, food, and cosmetics industry, to name just a few.

The punicic acid used in the described methods may be in a free acid form or bound chemically through ester linkages. In its natural form, punicic acid is heat stable. Punicic acid may be used in its natural oil state or in a dried and powdered form. Further, the free acid form of punicic acid may be converted into a non-toxic salt, such as sodium, potassium or calcium salts, by reacting chemically equivalent amounts of the free acid form with an alkali hydroxide at a basic pH.

### Administration

In the course of the method of the present invention, a therapeutically effective amount of punicic acid compound is administered to an animal, Including mammals and humans, in many ways. While in the preferred embodiment, the punicic acid compound is administered orally or parenterally, other forms of administration such as through medical compounds or aerosols are also contemplated.

For oral administration, the effective amount of punicic acid may be administered in, for example, a solid, semi-solid, liquid or gas state. Specific examples include tablet, capsule, powder, granule, solution, suspension, syrup, and elixir agents. However, the punicic acid compound is not limited to these forms.

To formulate the punicic acid of the present invention into tablets, capsules, powders, granules, solutions or suspensions, the punicic acid compound is preferably mixed with a binder, a disintegrating agent and/or a lubricant. If necessary, the resultant composition may be mixed with a diluent, a buffer, an infiltrating agent, a preservative and/or a flavor, using known methods. Examples of the binder include crystalline cellulose, cellulose derivatives, cornstarch, and gelatin. Examples of the disintegrating agent include cornstarch, potato starch, and sodium carboxymethylcellulose. Examples of the lubricant include talc and magnesium stearate. Further, additives, which have been conventionally used, such as lactose and mannitol, may also be used.

For parenteral administration, the punicic acid compound of the present invention may be administered rectally or by injection. For rectal administration, a suppository may be used. The suppository may be prepared by mixing the punicic acid of the present invention with a pharmaceutically suitable excipient that melts at body temperature but remains solid at room temperature. Examples include but are not limited to cacao butter, carbon wax or polyethylene glycol. The resulting composition may be molded into any desired form using methods known to the field.

For administration by injection, the punicic acid compound of the present invention may be injected hypodermically, intracutaneously, intravenously or intramuscularly. Medicinal drugs for such injection may be prepared by dissolving, suspending or emulsifying the punicic acid of the invention into an aqueous or non-aqueous solvent such as vegetable oil, glyceride of synthetic resin acid, ester of higher fatty acid, or propylene glycol by a known method. If desired, additives such as a solubilizing agent, an osmoregulating agent, an emulsifier, a stabilizer, or a preservative, which has been conventionally used may also be added.

For formulating the punicic acid of the present invention into suspensions, syrups or elixirs, a pharmaceutically suitable solvent may be used.

The punicic acid compound of the present invention may also be used together with an additional compound having other pharmaceutically suitable activity to prepare a medicinal drug.

The punicic acid of the present invention may also be administered in the form of an aerosol or inhalant prepared by charging the punicic acid in the form of a liquid or fine powder, together with a gaseous or liquid spraying agent and, if necessary, a known auxiliary agent such as an inflating agent, into a non-pressurized container such as an aerosol container or a nebulizer. A pressurized gas of, for example, dichlorofluoromethane, profane or nitrogen may be used as the spraying agent.

Punicic acid may be administered to an animal, including mammals and humans, in need thereof as a pharmaceutical or veterinary composition, such as tablets, capsules, solutions or emulsions. In a preferred embodiment of the invention, the free acid form of punicic acid is administered. However, administration of other forms of punicic acid, including but not limited to esters thereof, pharmaceutically-suitable salts thereof, metabolizes thereof, and combinations thereof, in a single dose or a multiple dose, are also contemplated by the present invention.

Punicic acid may also be administered to an animal in need thereof as a nutritional additive, either as a food or nutraceutical supplement.

The terms "preventing or treating" "treating or ameliorating" and similar terms used herein, include prophylaxis and full or partial treatment. The terms may also include reducing symptoms, ameliorating symptoms, reducing the severity of symptoms, reducing the incidence of the disease, or any other change in the condition of the patient, which improves the therapeutic outcome.

The punicic acid is preferably used and/or administered in the form of a composition. Suitable compositions are, preferably, a pharmaceutical composition, a foodstuff or a food supplement. These compositions provide a convenient form in which to deliver the punicic acid. Compositions of the invention may comprise an antioxidant in an amount effective to increase the stability of the punicic acid with respect to oxidation.

The amount of punicic acid that is administered In the method of the invention or that is for administration in the use of the invention is preferably from about 0.001g to about 20g (more preferably 0.1g to 10g, such as 0.5g to 5g) of punicic acid or derivative thereof per day. Suitable compositions can be formulated accordingly.

A preferred composition according to the invention is a foodstuff. Food products (which term includes animal feed) preferably contain a fat phase, wherein the fat phase contains punicic acid. The foodstuffs are optionally used as a blend with a complementary fat. For example, the blend may comprise 0.3 - 95 wt%, preferably 2-80 wt %, most preferably 5-40 wt% ofpunicic acid and 99.7 - 5 wt %, preferably 98-20 wt %, most preferably 95-60 wt % of a complementary fat, for example selected from: cocoa butter, cocoa butter equivalents, palm oil or fractions thereof, palmkernel oil or factions thereof, interesterified mixtures of said fats or fractions thereof, or liquid oils, selected from: sunflower oil, high oleic sunflower oil, soybean oil, rapeseed oil, cottonseed oil, fish oil, safflower oil, high oleic safflower oil, maize oil and MCT-oils. Examples of suitable foodstuffs include those selected from the group consisting of margarines, fat continuous or water continuous or bicontinuous spreads, fat reduced spreads, confectionery products such as chocolate or chocolate coatings or chocolate fillings or bakery fillings, ice creams, ice cream coatings, ice cream inclusions, dressings, mayonnaises, cheeses, cream alternatives, dry soups, drinks, cereal bars, sauces, snack bars, dairy products, clinical nutrition products and infant formulations.

Other examples of compositions are pharmaceutical compositions, such as in the form of tablets, pills, capsules, caplets, multiparticulates including: granules, beads, pellets and micro-encapsulated particles; powders, elixirs, syrups, suspensions and solutions. Pharmaceutical compositions will comprise a pharmaceutically acceptable diluent or carrier. Pharmaceutical compositions are preferably adapted for administration parenterally (e.g., orally). Orally administrable compositions may be in solid or liquid form and may take the form of tablets, powders, suspensions and syrups. Optionally, the compositions comprise one or more flavouring and/or colouring agents.

Pharmaceutically acceptable carriers suitable for use in such compositions are well known in the art of pharmacy. The compositions of the invention may contain 0.1-99% by weight of punicic acid. The compositions of the invention are generally prepared in unit dosage form. Preferably the unit dosage of punicic acid is from 1 mg to 1000mg (more preferably from 100mg to 750mg). The excipients used in the preparation of these compositions are the excipients known in the art.

Further examples of product forms for the composition are food supplements, such as in the form of a soft gel or a hard capsule comprising an encapsulating material selected from the group consisting of gelatin, starch, modified starch, starch derivatives such as glucose, sucrose, lactose and fructose. The encapsulating material may optionally contain cross-linking or polymerizing agents, stabilizers, antioxidants, light absorbing agents for protecting light-sensitive fills, preservatives and the like. Preferably, the unit dosage of punicic acid in the food supplements is from 1mg to 1000mg (more preferably from 100mg to 750mg).

The use of the present invention administers a therapeutically effective amount of punicic acid compound to an animal in need thereof. The effective amount of punicic acid depends on the form of punicic acid compound administered, the duration of the administration, the route of administration (e.g., oral or parenteral), the age of the animal and the condition ot the animal.

An amount of punicic acid effective to treat and prevent IBD, including UC and CD can range from 50 to 500 mg/kg/day, with a preferred dose of 100 to 150 mg/kg/day.

When the effective amount of the punicic acid compound of the present invention is administered in a nutritional, medical or veterinary composition, the preferred dose ranges from about 0.01 to 2.0% wt/wt to the food or nutraceutical product.

### Preparation of punicic acid

Punicic acid-enriched pomegranate oil has been previously isolated from pomegranate seeds and described as an anti-carcinogen (Kim et al. 2002; Hora et al. 2003). Pomegranate seeds were separated from their juice sacks, washed in water and dried in a convection dryer. Oil extrusion was performed by "cold press" at 80 °C, using a Type 40A electric screw press (Skeppsta Maskin, Orebro, Sweden). The resulting oil contained 480 mg of punicic acid per gram of oil. Punicic acid can also be obtained by enzymatic biosynthesis through methods known in the field. (Iwabuchi et al. 2003), (Hornung et al. 2002).

However, because enzymatic synthesis and cold press methods ofpunicic acid extraction are inefficient (e.g., 10% yield), solvent extraction methods are recommended. Before solvent extraction, seeds are steam-heated to reduce enzymatic hydrolysis and improving processing. After heating, seeds are finely ground and used in a solvent extraction. Regular liquid solvents such as hexane require soaking the seeds multiple times for up to 12 to 20 hours with stirring, filtering, and solvent evaporation. Alternatively, punicic acid-enriched pomegranate oil may be generated by CO₂ super critical extraction in methods known to the art.

Because the oil is more susceptible tn oxidative processes when released from the seed, extraction is preferably performed under a nitrogen blanket to prevent contact with the air. Additionally, the oil is nitrogen-purged and stored with one or various antioxidants in the dark at 4 °C or at -20 °C for longer-term storage. Oil from pomegranate seeds is expressed during this process in an amount by weight of 18% of the weight of the seeds.

The practice of the present invention is further illustrated by the following experiments, which were conducted.

The following example iillustrates the invention. In the example and throughout this specification, all percentages, parts and ratios are by weight unless indicated otherwise.

### EXPERIMENT

### Experiment 1

### Objective

To determine the effect of punicic acid on IBD and colitis-associated disease.

### Methods

in the first experiment, eleven (11) C57BL6 mice were fed zero or 0.6 g punicic acid /100 g food (1% pomegranate oil) for thirty-eight (38) days. The diets were made isocaloric hy replacing (wt/wt basis) punicic acid with linoleic acid in the control diet. On day 32 of the experiment (seven days prior to sacrificing the mice), intestinal inflammation was induced by challenging mice with 2.5% DSS, 36,000 to 44,000 mol wt (ICN Biomedicals, Aurora, OH) in the drinking water. Animal models of IBD, including the DSS colitis model represent a means to probe the immunological pathogenesis of IBD (Strober et al. 2002) and a safe method of testing the preventive and/or therapeutic efficacy of novel compounds, such as punicic acid. One of the key features of the DSS challenge is its ability to disrupt the epithelial cell barrier and promote increased cellular exposure to normal luminal and mucosal microflora (Strober et al. 2002). Following the DSS challenge, mice were weighed on a daily basis and examined for clinical signs of disease associated with colitis (i.e., perianal) soiling, rectal bleeding, diarrhea, and piloerection) by blinded observers. The disease activity indices and rectal bleeding scores were calculated using a previously published compounded clinical score (Saubermann et al. 2000; Bassaganya-Riern et al. 2004).

After the mice were sacrificed, the colons, brains, kidneys, livers and spleens were harvested from the mice. The colons were weighed and scored by blinded observers based on the severity of macroscopic lesions following a scale on 0 to 3 (e.g., 0 = no lesions and 3 = severe lesions). Colonic contents were washed by gently rinsing the colon with sterile 1 X PBS using a mouse gavage needle connected to a 5 mL syringe. The other tissues were directly added to the formalin beaker. All specimens were generally labeled with the following information: 1) mouse number; 2) date collected; 3) experiment number; 4) type of solvent; and 5) tissue type. Sections of colon, brain, kidney and liver were fixed in 10% buffered neutral formalin (Fisher, Atlanta, GA), later embedded in paraffin, and then sectioned (6-µm) and stained with the hematoxylin and eosin stain (H&E) (AML Labs, Baltimore, MD) for histological examination. Tissue slides were examined in an Olympus microscope (Olympus America Inc., Dulles, VA) and processed in Adobe Photoshop Elements 2.0 (Adobe Systems Inc., San Jose, CA).

Colons were graded with a compounded histological score including the extent of 1) crypt damage and 2) regeneration, 3) metaplasia/hyperplasia, 4) lamina proprial vascular changes, 5) submucosal changes and 6) presence of inflammatory infiltrates. The sections were graded with a range from 0 to 4 for each of the previous categories and data was presented as a normalized compounded score. Briefly, crypt damage: 0 = none; 1 = basal 1/3; 2 = basal 2/3; 3 = only surface epithelium intact; 4 = entire crypt and epithelium lost. For epithelial erosion: 0 = no erosion; 1 = mild focal erosion; 2 = mild multifocal erosion; 3 = significant ulcers throughout the colon; 4 = colonic architecture lost. For mucosal thickness: 0 = normal thickness; 1 = mild increase in thickness; 2 - mild to severe increase in thickness; 3 = severe increase in thickness; 4 = mucosa obliterating the lumen of the intestine. For regeneration: 4 = no tissue repair; 3 = surface epithelium not intact; 2 = regeneration with crypt depletion; 1 = almost complete regeneration; 0 = complete regeneration or normal tissue. For metaplasia/hyperplasia: 0 = none; 1 = mild goblet cell; 2 = severe goblet cell metaplasia; 3 - goblet cell metaplasia and presence of immature cells in the base and extending to 2/3 of the gland; 4 = presence of immature cells in the base and extending beyond 2/3 of the gland. For vascular changes: 0 = none; 1 = mild capillary dilation in the lamina propria; 2 = mild capillary dilation in the lamina propria and submucosa; 3 = severe capillary dilation; 4 = lamina proprial hemorrhage. For lamina proprial leukocytic infiltrates: 0 = normal infiltrate (equal matrix of plasma cells and lymphocytes with some neutrophils); 1 = mild increase of neutrophils in lamina propria; 2 = mild increase in neutrophils extending towards the submucosa; 3 = severe neutrophil homing in the lamina propria and/or submucosa; 4 = severe neutrophil homing in the lamina propria and/or submucosa and plasmacytic shift of the lamina propria. Brain, kidney and liver were collected to assess the safety of oral punicic acid administration.

Statistical analysis was performed by analysis of variance (ANOVA). The ANOVA was performed using the general linear model procedure of SAS (SAS 1988) as previously described (Hassaganya-Riera et al. 2003). Differences with probability value (P<0.05) were considered statistically significant.

### Results

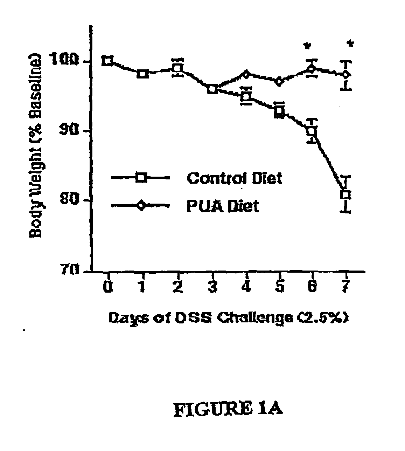

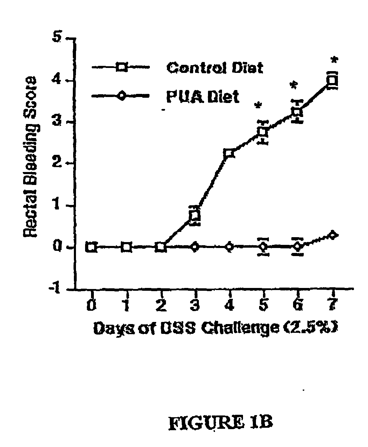

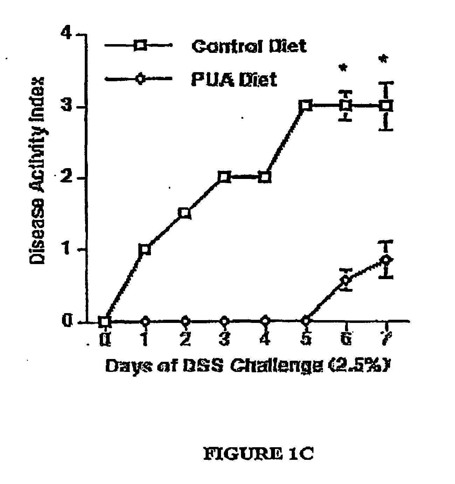

The mortality associated with colonic inflammation in the control group was 50% by day 6 of the 2.5% DSS challenge, whereas the mortality rate in punicic acid-fed mice was 0%. The average onset of clinical disease in control mice was 1 day after initiating the DSS challenge; whereas in mice fed the control diet, clinical disease did not appear until 6 days after the DSS challenge. On average, rectal bleeding started on day 4 of the DSS challenge in control-fed mice, whereas it did not start until day 7 of the DSS challenge in mice fed punicic acid-supplemented diets. The DSS challenge induced weight losses, colitis-associated clinical disease and rectal bleeding, which appeared earlier and were more severe in mice fed the control diet than in those fed punicic acid-supplemented diets. Figure 1A illustrates the effects of dietary punicic acid (0.6 g/100g) and an isocaloric control dict on body weight losses during a 7-day DSS challenge (2.5%, wt/vol), which is representative of experimentally induced IBD. Figure 1B illustrates the effects of dietary punicic acid (0.6 g/100g) and an isocaloric control diet on rectal bleeding during a 7-day DSS challenge (2.5%, wt/vol). Figure 1C illustrates the effects of dietary punicic acid (0.6 g/100g) and an isocaloric control diet on disease activity indices during a 7-day DSS challenge (2.5%, wt/vol). Mice were fed a punicic acid-supplemented diet for 32 days prior to the DSS challenge. Colitis-associated disease was improved. The prevention or amelioration of clinical signs of colonic inflammation correlated with lower colon weights in punicic acid-fed mice and less severe scores upon macroscopic analysis of lesions.

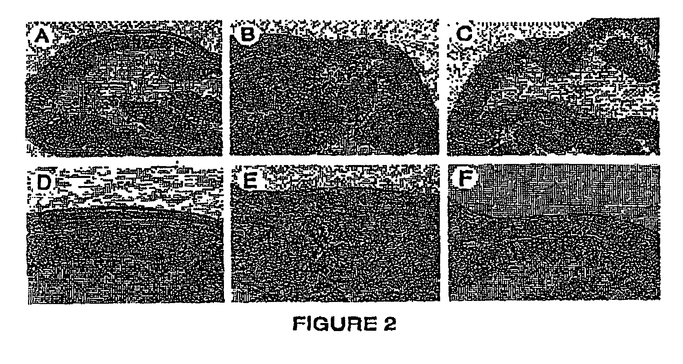

Specifically, following the DSS challenge, the average macroscopic score (in a scale from 0 to 3) in colons of mice fed the control diet was 3 whereas in punicic acid-fed mice the score was 1.4. The average colon weights in control-fed mice were 0.958 grams whereas those of punicic acid-fed mice averaged 0.153 grams. These lower colon weights and milder lesions can be caused by decreased inflammatory cell migration into the colon. Histopathological examination of the H&E-stained slides of colon under the microscope revealed no major lesions in punicic acid fed mice, whereas mice fed the control diet presented thickening of the colonic mucosa, epithelial erosion, and infiltration of inflammatory cells. At 20 X, a loss of the epithelial surface was observed in Figure 2C when compared with Figure 2A. At a magnification of 40 X, Figures 2E and 2F showed inflammatory cell infiltration, flattening of epithelial cells and epithelial erosion. Figure 2B showed a thickening of the column mucosa without erosion or infiltration. Figure 2D showed the epithelial cells maintained their normal columnar shape.

The scoring of the pathological findings reveals that the severity of colonic lesions in control-fed mice was greater than in punicic acid-fed mice (Table 1).

**Table 1. Histological scores following a 7-day challenge period with 2.5% DSS.**

| Item | Control Diet | PUNICIC ACID diet | *P* value |
|---|---|---|---|
| Mucosal | 4.00±0.42* | 1.83±0.24 | 0.0146 |
| Thickness | | | |
| Epithelial Erosion | 4.00±0.12* | 0.00±0.00 | 0.0001 |
| Inflammatory | 4.00±0.30* | 0.50±0.17 | 0.0001 |
| Cell Infiltration | | | |
| Vascular Changes | 0.50±0.20 | 0.50±0.35 | 0.1927 |
| Metaplasia/ | 3.50±0.50* | 0.50±0.28 | 0.0061 |
| Hyperplasia | | | |
| Crypt Damage | 4.00±0.45 | 0.33±0.26 | 0.0009 |

| | | | |
|---|---|---|---|
| Values are least square means ± standard error of the mean. * Illustrates that the value is statistically different. | | | |

It is generally accepted that the goats of IBD preventive or therapeutic approaches include inducing substantial clinical improvements (Lichtenstein et al. 2003). The oral administration of punicic acid resulted in significant clinical and pathological improvements. In addition, the macroscopic and histologic (data not shown) analysis of key organs involved in metabolism (i.e., liver and kidneys) and the brain revealed no abnormalities, which suggests that the dose utilized was effective and safe. No adverse effects were observed throughout the duration of the study.

### BIBLIOGRAPHY

Bassaganya-Riera, J., R. Hontecillas, et al. (2001). "Effects of dictary conjugated linoleic acid in nursery pigs ofdirty and clean environments on growth, empty body composition, and immune competence." J Anim Sci 79(3); 7.54-21.
Bassaganya-Riera, J.. R. Hontecillas, et al. (2001). "Dietary conjugated linoleic acid modulates phenotype and effector functions of porcine cd8(+) lymphocytes." J Nutr 131(9): 2370-7.
Bassaganya-Riera, J., R. Hontecillas, et al. (2002). "Long-term influence of lipid nutrition on the induction of CD8(+) responses to viral or bacterial antigens." Vaccine 20(9-10): 1435-44.
Bassaganya-Riera, J., R. M. Pogranichniy, et al. (2003). "Conjugated Linoleic Acid Ameliorates Viral Infectivity in a Pig Model of Virally Induced Immunosuppression." J Nutr 133: 3204-3214.
Bassaganya-Riera, J., K. Reynolds, et al. (2004). "Activation of PPAR gamma and delta by conjugated linoleic acid mediates protection from experimental inflammatory bowel disease," Gastroenterology 127(3): 777-91.
Camilleri, M. (2003). "GI clinical research 2002-2003: The year in review." Clinical Gastroenterology and Hepatology 1: 415-420.
Hora, J. J., E. R. Maydew, et al. (2003). "Chemoproventive effects of pomegranate seed oil on skin tumor development in CD1 mice." J Med Food 6(3): 157-61.
Hornung, E., C. Pernstich, et al. (2002). "Formation of conjugated Delta11Delta13-double bonds by Delta12-linoleic acid (1,4)-acyl-lipid-desaturase in pomegranate seeds." Eur J Biochem 269(19): 4852-9.
Iwabuchi, M., J. Kohno-Murase, et al. (2003). "Delta 12-oleate desaturase-related enzymes associated with formation of conjugated trans-delta 11, cis-delta 13 double bonds." J Biol Chem 278(7): 4603-10.
Kim, N. D., R. Mehta, et al. (2002). "Chemopreventive and adjuvant therapetuic potential of pomegranate (Punica granatum) for human breast cancer." Breast Cancer Research and Treatment 71: 203-207.
Lichtenstein, G. R., M. Abreu, et al. (2003). Recent advances in the treatment of Crohn's colitis, The center for health care education, LLC.
Moller, D. E. and J. P. Berger (2003). "Role of PPARs in the regulation of obesity-related insulin sensitivity and inflammation. Int J Obes Relat Metab Disord 27 Suppl 3: S17-21.
Rubins, H. B. and S. J. Robins (2000). "Conclusions from the VA-HIT study." Am J Cardiol 86(5): 543-4.
SAS (1988). SAS/STAT User's guide (Release 6.0.3). Cary, NC, SAS Inst. Inc.
Saubermann, L. J., P. Beck, et al. (2000). "Activation of natural killer T cells by alpha-galactosylceramide in the presence of CD1d provides protection against colitis in mice." Gastroenterology 119(1): 119-28.
Strober, W., I. J. Fuss, et al. (2002). "The immunology of mucosal models of inflammation." Annu Rev Immunol 20: 495-549.
Vohl, M. C., R. Sladek, et al. (2004). "A survey of genes differentially expressed in subcutaneous and visceral adipose tissue in men." Obes Res 12(8): 1217-22.

## Claims

1. Punicic acid , esters thereof or pharmaceutically suitable salts thereof for use for treating, preventing or ameliorating inflammatory bowel disease.

2. Punicic acid, esters thereof or pharmaceutically suitable salts thereof for use according to claim 1, wherein the inflammatory bowel disease is Crohn'disease.

3. Punicic acid, esters thereof or pharmaceutically suitable salts thereof for use according to claim 1, wherein the inflammatory bowel disease is ulcerative colitis.

4. Punicic acid , esters thereof or pharmaceutically suitable salts thereof for use according to claim 1, wherein the punicic compound is administered orally.

5. Punicic acid, esters thereof or pharmaceutically suitable salts thereof for use according to claim 4, wherein the punicic compound is administered alone or in combination with a pharmaceutically suitable carrier.

6. Punicic acid, esters thereof or pharmaceutically suitable salts thereof for use according to claim 1, wherein the punicic compound is administered parenterally.

7. Punicic acid, esters thereof or pharmaceutically suitable salts thereof for use according to claim 6, wherein the punicic compound is administered alone or in combination with a pharmaceutically suitable carrier.

8. Punicic acid, esters thereof or pharmaceutically suitable salts thereof for use according to claim 1 to 3, , wherein the punicic compound is administered in combination with a nutritional food supplement.

9. Punicic acid, esters thereof or pharmaceutically suitable salts thereof for use according to claim 1 to 3, wherein the punicic compound is administered in combination with other active ingredients such as vitamins or other fatty acids.

10. Use of punicic acid, esters thereof or pharmaceutically suitable salts thereof to prepare a medicament for treating, preventing or ameliorating inflammatory bowel disease.

11. Use according to claim 10, wherein the inflammatory bowel disease is Crohn'disease.

12. Use according to claim 10, wherein the inflammatory bowel disease is ulcerative colitis.

13. Use according to claim 10, wherein the punicic compound is administered orally.

14. Use according to claim 13, wherein the punicic compound is administered alone or in combination with a pharmaceutically suitable carrier.

15. Use according to claim 10, wherein the punicic compound is administered parenterally.

16. Use according to claim 15, wherein the punicic compound is administered alone or in combination with a pharmaceutically suitable excipient.

17. Use of punicic acid, esters thereof or pharmaceutically suitable salts thereof according to claim 10 to 13, wherein the punicic compound is administered in combination with a nutritional food supplement.

18. Use according to claim 10 to 13, wherein the punicic compound is administered in combination with other active ingredients such as vitamins or other fatty acids.

## Patentansprüche

1. Punicinsäure, ihre Ester oder ihre pharmazeutisch geeigneten Salze zur Verwendung zur Behandlung, Vorbeugung oder Linderung entzündlicher Darmerkrankungen.

2. Punicinsäure, ihre Ester oder ihre pharmazeutisch geeigneten Salze zur Verwendung gemäß Anspruch 1, wobei es sich bei der entzündlichen Darmerkrankung um Morbus Crohn handelt.

3. Punicinsäure, ihre Ester oder ihre pharmazeutisch geeigneten Salze zur Verwendung gemäß Anspruch 1, wobei es sich bei der entzündlichen Darmerkrankung um Colitis ulcerosa handelt.

4. Punicinsäure, ihre Ester oder ihre pharmazeutisch geeigneten Salze zur Verwendung gemäß Anspruch 1, wobei die Punicinverbindung oral verabreicht wird.

5. Punicinsäure, ihre Ester oder ihre pharmazeutisch geeigneten Salze zur Verwendung gemäß Anspruch 4, wobei die Punicinverbindung pur oder in Verbindung mit einem pharmazeutisch geeigneten Trägerstoff verabreicht wird.

6. Punicinsäure, ihre Ester oder ihre pharmazeutisch geeigneten Salze zur Verwendung gemäß Anspruch 1, wobei die Punicinverbindung parenteral verabreicht wird.

7. Punicinsäure, ihre Ester oder ihre pharmazeutisch geeigneten Salze zur Verwendung gemäß Anspruch 6, wobei die Punicinverbindung pur oder in Verbindung mit einem pharmazeutisch geeigneten Trägerstoff verabreicht wird.

8. Punicinsäure, ihre Ester oder ihre pharmazeutisch geeigneten Salze zur Verwendung gemäß Anspruch 1 bis 3, wobei die Punicinverbindung in Verbindung mit einem Nahrungsergänzungsmittel verabreicht wird.

9. Punicinsäure, ihre Ester oder ihre pharmazeutisch geeigneten Salze zur Verwendung gemäß Anspruch 1 bis 3, wobei die Punicinverbindung in Verbindung mit anderen Wirkstoffen wie Vitaminen oder anderen Fettsäuren verabreicht wird.

10. Punicinsäure, ihre Ester oder ihre pharmazeutisch geeigneten Salze zur Zubereitung eines Medikaments zur Behandlung, Vorbeugung oder Linderung entzündlicher Darmerkrankungen.

11. Verwendung gemäß Anspruch 10, wobei es sich bei der entzündlichen Darmerkrankung um Morbus Crohn handelt.

12. Verwendung gemäß Anspruch 10, wobei es sich bei der entzündlichen Darmerkrankung um Colitis ulcerosa handelt.

13. Verwendung gemäß Anspruch 10, wobei die Punicinverbindung oral verabreicht wird.

14. Verwendung gemäß Anspruch 13, wobei die Punicinverbindung pur oder in Verbindung mit einem pharmazeutisch geeigneten Trägerstoff verabreicht wird.

15. Verwendung gemäß Anspruch 10, wobei die Punicinverbindung parenteral verabreicht wird.

16. Verwendung gemäß Anspruch 15, wobei die Punicinverbindung pur oder in Verbindung mit einem pharmazeutisch geeigneten Hilfsstoff verabreicht wird.

17. Verwendung der Punicinsäure, ihrer Ester oder ihrer pharmazeutisch geeigneten Salze gemäß Anspruch 10 bis 13, wobei die Punicinverbindung in Verbindung mit einem Nahrungsergänzungsmittel verabreicht wird.

18. Verwendung gemäß Anspruch 10 bis 13, wobei die Punicinverbindung in Verbindung mit anderen Wirkstoffen wie Vitaminen oder anderen Fettsäuren verabreicht wird.

## Revendications

1. Acide punicique, esters d'acide punicique ou sels d'acide punicique pharmaceutiquement acceptables, destinés au traitement, à la prévention et au soulagement des maladies inflammatoires de l'intestin.

2. Acide punicique, esters d'acide punicique ou sels d'acide punicique pharmaceutiquement acceptables, destinés à une utilisation selon la revendication 1, lorsque la maladie inflammatoire de l'intestin est la maladie de Crohn.

3. Acide punicique, esters d'acide punicique ou sels d'acide punicique pharmaceutiquement acceptables, destinés à une utilisation selon la revendication 1, lorsque la maladie inflammatoire de l'intestin est la colite ulcéreuse.

4. Acide punicique, esters d'acide punicique ou sels d'acide punicique pharmaceutiquement acceptables, destinés à une utilisation selon la revendication 1, **caractérisée en ce que** le composé punicique est administré par voie orale.

5. Acide punicique, esters d'acide punicique ou sels d'acide punicique pharmaceutiquement acceptables, destinés à une utilisation selon la revendication 1, **caractérisé en ce que** le composé punicique est administré seul ou en combinaison avec un vecteur pharmaceutiquement acceptable.

6. Acide punicique, esters d'acide punicique ou sels d'acide punicique pharmaceutiquement acceptables, destinés à une utilisation selon la revendication 1, **caractérisé en ce que** le composé punicique est administré par voie parentérale.

7. Acide punicique, esters d'acide punicique ou sels d'acide punicique pharmaceutiquement acceptables, destinés à une utilisation selon la revendication 6, **caractérisée en ce que** le composé punicique est administré seul ou en combinaison avec un vecteur pharmaceutiquement acceptable.

8. Acide punicique, esters d'acide punicique ou sels d'acide punicique pharmaceutiquement acceptables, destinés à une utilisation selon les revendications 1 à 3, **caractérisée en ce que** le composé punicique est administré en combinaison avec un complément alimentaire.

9. Acide punicique, esters d'acide punicique ou sels d'acide punicique pharmaceutiquement acceptables, destinés à une utilisation selon les revendications 1 à 3, **caractérisée en ce que** le composé punicique est administré en combinaison avec d'autres principes actifs tels que des vitamines ou d'autres acides gras.

10. Utilisation d'acide punicique, d'esters d'acide punicique ou de sels d'acide punicique pharmaceutiquement acceptables, dans l'élaboration d'un médicament destiné au traitement, à la prévention et au soulagement des maladies inflammatoires de l'intestin.

11. Utilisation selon la revendication 10, lorsque la maladie inflammatoire de l'intestin est la maladie de Crohn.

12. Utilisation selon la revendication 10, lorsque la maladie inflammatoire de l'intestin est la colite ulcéreuse.

13. Utilisation selon la revendication 10, **caractérisé en ce que** le composé punicique est administré par voie orale.

14. Utilisation selon la revendication 1 **caractérisée en ce que** le composé punicique est administré seul ou en combinaison avec un vecteur pharmaceutiquement acceptable.

15. Utilisation selon la revendication 10, **caractérisée en ce que** le composé punicique est administré par voie parentérale.

16. Utilisation selon la revendication 10, **caractérisée en ce que** le composé punicique est administré seul ou en combinaison avec un excipient pharmaceutiquement acceptable.

17. Utilisation d'acide punicique, d'esters d'acide punicique ou de sels d'acide punicique pharmaceutiquement acceptables, destinés à une utilisation selon les revendications 10 à 13, **caractérisée en ce que** le composé punicique est administré en combinaison avec un complément alimentaire.

18. Utilisation selon les revendications 10 à 13, **caractérisée en ce que** le composé punicique est administré en combinaison avec d'autres principes actifs tels que des vitamines ou d'autres acides gras.
